# EUROPEAN PATENT APPLICATION

(11) **EP 1 900 385 A1**
(43) Date of publication of application: **19.03.2008**
(21) Application number: 05759535.7
(22) Date of filing: 01.07.2005
(51) Int. Cl.: A61M 5/158, A61M 5/32, B21G 1/08

(54) **A DISPOSABLE NEEDLE FOR SYRINGES AND INFUSIONS AND THE MANUFACTURE THEREOF**

(30) Priority: 20.06.2005 CN 200510076875
(71) Applicant: Guo, Yuxian, Lvyuan District Changchun Jilin 130062 (CN)
(72) Inventor: Guo, Yuxian, Lvyuan District Changchun Jilin 130062 (CN)
(74) Representative: Müller, Karl-Ernst
(86) International application number: PCT/CN2005/000962
(87) International publication number: WO 2006/136062

(57) **Abstract**

A disposable needle for syringes and infusions includes a tubular needle body (1), one end of which consists of a pyramid needle tip (2), a conical needle tip (4) or the combination of a pyramid needle tip and a conical needle tip, a through-hole(3) communicating with the inner hole of the needle body (1) is provided on the tubular needle body (1) and the conical needle tip (4), or on the other part. The said conical needle tip (4) is solid, hollow or locally hollow. A manufacture of the said needle is also provided.

## Description

### Technical Field

The present invention relates to an iatrical apparatus, more particularly a disposable needle for syringes and infusions and the manufacture thereof.

### Background Art

The conventional needle for injection generally consists of tubular needle body and needle base coupled with one end of the needle body, in which the needle body with tube of slender and thin wall is grinded at the fore end thereof into a taper surface so as to form a needle tip. This type of needle tip is also formed as a blade due to the interior wall and taper surface of the rear part of needle tip so as to cut off the skin and flesh tissue. Therefore, the conventional needle for syringes will cut off a piece of skin and flesh tissue with the diameter same as the inner diameter of needle and then send it into the body so as to make hurt to human body. For the injection to muscle, a micro-hole could be left on injected part after needle pulled out which can be apparent that it is possible to be infection and bleeding. If the medicine is injected through blood vessel, it is possible to take a piece of skin and flesh tissue into the blood vessel so as to make great hurt to human body.

In order to solve above problems, patent application No. ZL01241577 has disclosed an injection needle which is primarily characterized in that the needle tip is pyramid shape, the groove root of which is provided with liquid medicine through-holes. The shortcoming is in that the liquid medicine through-holes are defined in the groove root of the pyramid shape. Since the pyramid surfaces of pyramid shape are still taper surface, the above problems are present in the liquid medicine through-holes with taper surfaces in a certain degree.

### Content of invention

The object of the invention is to provide a disposable needle for syringes and infusions in order to solve the problem in conventional art that during using it will cut off a piece of skin and flesh tissue with the diameter same as the inner diameter of needle and then send it into the body so as to make hurt to human body. The present invention also provides a method for manufacturing above said disposable needle for syringes and infusions.

The technical project of the invention is as follows:

A disposable needle for syringes and infusions includes a tubular needle body.

One end of the said tubular needle body consists of pyramid, conical needle tip or the combination of pyramid and conical and liquid medicine through-holes communicating with the inner hole of the needle body are provided in the tubular needle body and the pyramid or conical needle tip.

The liquid medicine through-holes are provided in one or more pyramid or conical surfaces of the said pyramid or conical needle tip.

The said liquid medicine through-holes are arranged in other parts of the tubular needle body at the position adjacent to the needle tip, and the said pyramid or conical needle tip is solid, hollow or partially hollow.

A smoothly transient is provided between the said pyramid or conical needle tip and the tubular needle body.

The said pyramid surface and the conical surface can be concave or convex in the axial direction and the said liquid medicine through-holes are arranged in the fore part of the concave pyramid or conical surface or the rear part of the convex pyramid or conical surface; the said pyramid surface and conical surface can also be concave or convex in circumference direction, as well as both in axial and circumference directions.

A needle base which forms a complete set with the output port of syringes and infusions is coupled to the end of tubular needle body opposite to the needle tip.

A method for manufacturing the disposable needle for syringes and infusions includes steps of rolling tube, welding the seam, drawing and cutting. It further comprises the steps of forming, polishing and perforating for the needle tip after the cutting step.

An argon welder is utilized to weld the seam of the rolled tube into a flat in the said step of welding the seam, a drawing machine is utilized to draw the welded tube into tubular product required for syringes and infusions in the said step of drawing, and a cutting machine is utilized to cut the tubular product into required length in the said step of cutting.

The steps of forming, polishing and perforating for the needle tip are all performed in molding machine and comprise: the workpiece cut in cutting step is carried into the hopper of the molding machine; the workpiece is carried into mouth of tongs by the rotating grooved pulley in the molding machine; the redundant part for the workpiece modeling is cut by pairs of opposite close cutleries in the mouth of tongs; one end of the workpiece is then extruded into required pyramid needle tip by the multi-functional mold of the molding machine while the thickness of the needle tip part is ensured as the same as that of the tubular part (a special measure is needed to be described); the workpiece is clamped by the disk of the molding machine to rotate a angle of 45°; the workpiece is carried into the emery wheel oscillator to perform the step of polishing; and the workpiece is carried into the perforator to perform the step of perforating so as to form liquid medicine through-holes. The present invention has several advantages including: since the included angle between the fore part of concave pyramid or conical surface or rear part of convex pyramid or conical surface of pyramid or conical needle in axial direction and axial direction is very small, the radial dimension of the liquid medicine through-holes arranged in corresponding position is very small, which can further significantly reduce the possibility to hurt the body tissue due to the needle holes, The liquid medicine through-holes arranged in the tubular body adjacent to pyramid or conical needle tip avoids the possible hurt to body tissue due to the needle hole. Additionally, conical needle tip do not tend to hurt the body tissue during rotating in body, which is particularly apparent to the needle for infusions with large diameter.

### Description of Drawings

Figure 1 illustrates a structural view of three-edges-pyramid needle according to an embodiment of the invention, in which figure b shows the view of needle tip in figure a;
Figure 2 illustrates a structural view of three-edges-pyramid needle according to another embodiment of the invention, in which figure b shows the view of needle tip in figure a and figure c shows a cross-section view along the line A -Ain figure a;
Figure 3 illustrates a structural view of conical needle according to an embodiment of the invention, in which figure b shows the view of needle tip in figure a;
Figure 4 illustrates a structural view of conical needle according to another embodiment of the invention, in which figure b shows the view of needle tip in figure a;
Figure 5 illustrates a structural view of needle with combination of two-edges-pyramid and conical according to an embodiment of the invention, in which figure b shows the view of needle tip in figure a;
Figure 6 illustrates a structural view of conical needle with needle base according to an embodiment of the invention.

### Best Mode to Carrying Out the Invention

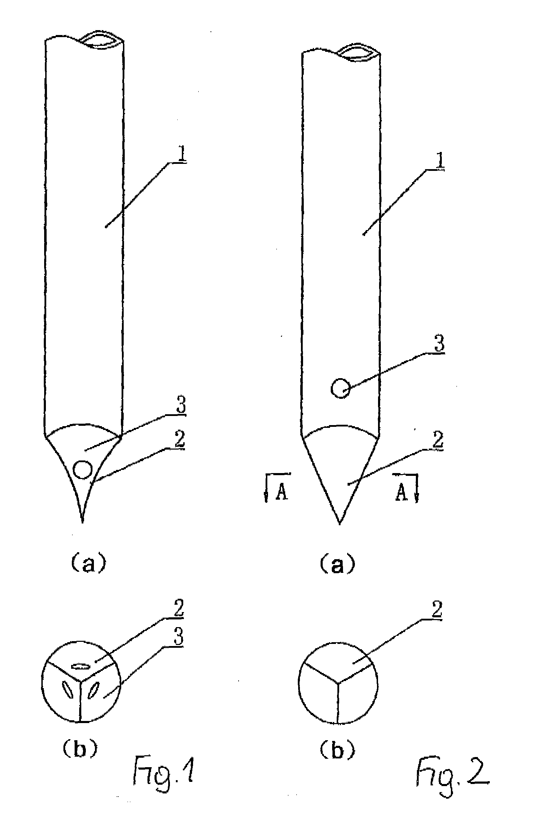

See figures 1 and 2, in which one end of the tubular needle body 1 of the embodiment has a three-edges-pyramid needle tip of which each pyramid surface 2 is slightly concave in axial and circumference directions and provided with a liquid medicine through-hole 3 adjacent to tips respectively thereon (as shown in figure 1). It can be seen from the figure 1b that the dimension of the liquid medicine through-hole 3 in radial direction is very small, The liquid medicine through-holes 3 can also be arranged in the tubular needle body 1 at the position adjacent to the needle tip (as shown in figure 2).

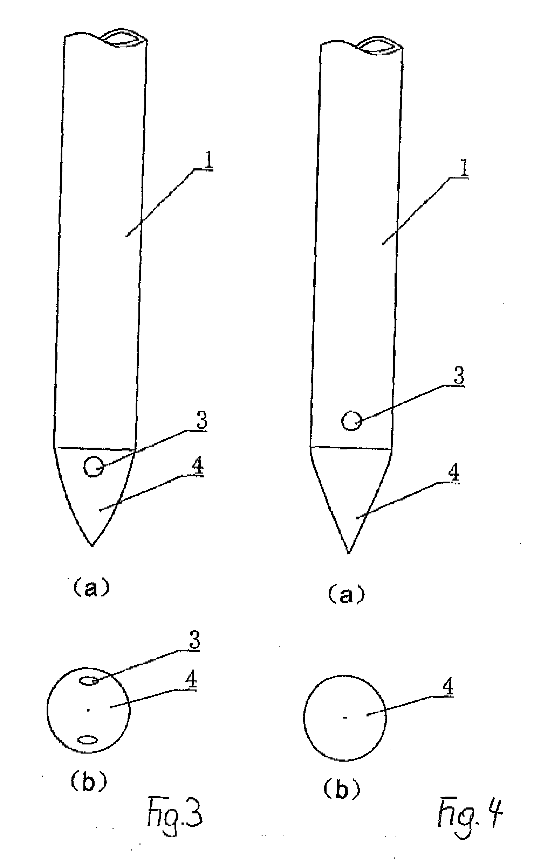

See figures 3 and 4, in which one end of the tubular needle body 1 of the embodiment has a conical needle tip of which the conical surface 4 is convex and provided with two liquid medicine through-holes 3 at the position adjacent to the tubular needle body 1 (as shown in figure 3). The number of liquid medicine through-holes 3 can be increased or decreased based on the application status. The liquid medicine through-holes 3 can also be arranged in the tubular needle body 1 at the position adjacent to the needle tip (as shown in figure 4).

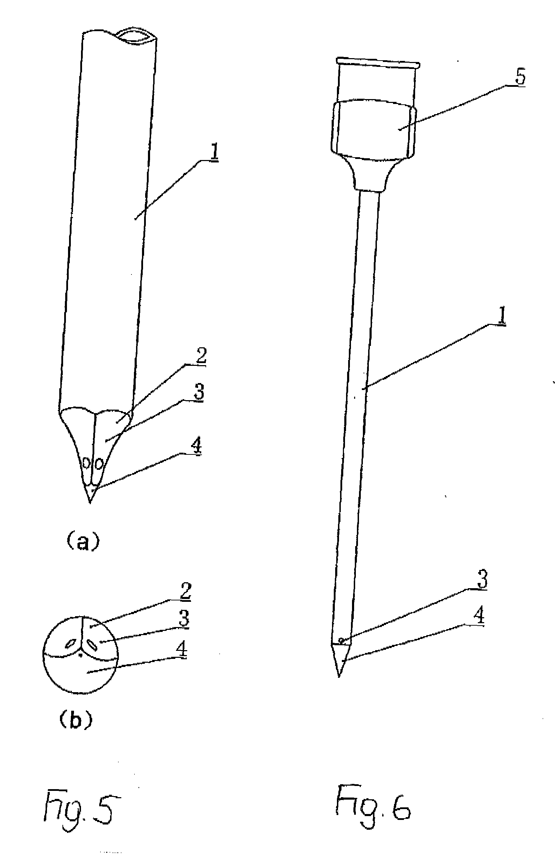

See figures 5, in which one end of the tubular needle body 1 of the embodiment has a needle consisted of the combination of two-edges-pyramid 2 and partially conical 4, in which the two pyramid surface 2 are concave in axial direction and provided with a liquid medicine through-hole 3 adjacent to tips respectively. The liquid medicine through-holes 3 also can be arranged in the conical part or the tubular needle body 1 at the position adjacent to the needle tip of the tubular needle body 1 (not shown).

See figures 6, which illustrates a structural view of embodiment with needle base according to the invention. Another end of the tubular needle body 1 according to the invention can be all coupled with a needle base 5. The needle base 5 contains various needle bases which form a complete set with syringes and infusions or the like.

The pyramid or conical needle tip of the invention is solid (in the case of the liquid medicine through-holes 3 arranged in the tubular needle body 1), hollow or partially hollow (in the case of the liquid medicine through-holes 3 arranged in the needle tip).

A smoothly transient is provided between the said pyramid 2 or conical needle tip 4 and the tubular needle body 1.

The said pyramid 2 contains regular or irregular multi-pyramid (i.e. the case that each of the pyramid surfaces has the same or different shape and dimension with each other). The said pyramid surface 2 and the conical surface 4 can be concave or convex in the axial direction (as shown in figure 3a). The said liquid medicine through-holes 3 are arranged in the fore part of the concave pyramid or conical surface or the rear part of the convex pyramid or conical surface, in which the dimensions of the liquid medicine through-holes 3 in axial direction are set as small as possible so as to reduce the possibility of injury to body tissue due to the needle hole. The said pyramid surface and conical surface can also be concave (as shown in figure 2c) or convex in circumference direction, as well as both in axial and circumference directions. It can also be concave or convex in circumference direction, as well as both in axial and circumference directions.

The technologic process of manufacture method according to the embodiment of the invention is: a stainless steel strap is rolled into tube; an argon welder is utilized to weld the seam of the rolled tube into a flat; a drawing machine is utilized to draw the welded tube into tubular product required for syringes and infusions; a cutting machine is utilized to cut the tubular product into required length; and perform steps of forming, polishing and perforating for the needle tip in molding machine. The steps of forming, polishing and perforating for the needle tip include: the workpiece cut in cutting step is carried into the hopper of the molding machine (which has been applied separately for patent with the application No. 200520007230 and titled a new manufacture machine for needle of syringe"); the workpiece is carried into mouth of tongs by the rotating grooved pulley in the molding machine; the redundant part for the workpiece modeling is cut by pairs of opposite dose cutleries in the mouth of tongs; one end of the workpiece is then extruded into required pyramid needle tip by the multi-functional mold of the molding machine while the thickness of the needle tip part is ensured as the same as that of the tubular part; the workpiece is clamped by the disk of the molding machine to rotate a angle of 45°; the workpiece is carried into the emery wheel oscillator to perform the step of polishing; and the workpiece is carried into the perforator to perform the step of perforating so as to form liquid medicine through-hole.

## Claims

1. A disposable needle for syringes and infusions including a tubular needle body, **characterized in that** one end of the said tubular needle body consists of pyramid, conical needle tip or the combination of pyramid and conical, and liquid medicine through-holes communicating with the inner hole of the needle body are provided in the tubular needle body and the pyramid or conical needle tip.

2. The disposal needle for syringes and infusions in claim 1, **characterized in that** the liquid medicine through-holes are provided in one or more pyramid or conical surfaces of the said pyramid or conical needle tip.

3. The disposable needle for syringes and infusions in claim 1, **characterized in that** the said liquid medicine through-holes are arranged in other parts of the tubular needle body at the position adjacent to the needle tip, and the said pyramid or conical needle tip is solid, hollow or partially hollow.

4. The disposable needle for syringes and infusions in claim 1, **characterized in that** a smoothly transient is provided between the said pyramid or conical needle tip and the tubular needle body.

5. The disposable needle for syringes and infusions in claim 1 , 2, 3, or 4, **characterized in that** the said pyramid surface and the conical surface can be concave or convex in the axial direction and the said liquid medicine through-holes are arranged in the fore part of the concave pyramid surface or the rear part of the convex pyramid or conical surface; the said pyramid surface and conical surface can also be concave or convex in circumference direction, as well as both in axial and circumference directions.

6. The disposable needle for syringes and infusions in claim 5, **characterized in that** a needle base which forms a complete set with the output port of syringes and infusions is coupled to the end of tubular needle body opposite to the needle tip.

7. A method for manufacturing the disposable needle for syringes and infusions in any one of Claims 1-6, including steps of rolling tube, welding the seam, drawing and cutting, **characterized in that** it further comprises the steps of forming, polishing and perforating for the needle tip after the cutting step.

8. The manufacture method in claim 7, **characterized in that** an argon welder is utilized to weld the seam of the rolled tube into a flat in the said step of welding the seam, a drawing machine is utilized to draw the welded tube into tubular product required for syringes and infusions in the said step of drawing, and a cutting machine is utilized to cut the tubular product into required length in the said step of cutting.

9. The manufacture method in claim 7 or 8. **characterized in that** the steps of forming, polishing and perforating for the needle tip are all performed in molding machine and comprise: the workpiece cut in cutting step is carried into the hopper of the molding machine; the workpiece is carried into mouth of tongs by the rotating grooved pulley in the molding machine; the redundant part for the workpiece modeling is cut by pairs of opposite close cutleries in the mouth of tongs; one end of the workpiece is then extruded into required pyramid needle tip by the multi-functional mold of the molding machine while the thickness of the needle tip part is ensured as the same as that of the tubular part (a special measure is needed to be described); the workpiece is clamped by the disk of the molding machine to rotate a angle of 45°; the workpiece is carried into the emery wheel oscillator to perform the step of polishing; and the workpiece is carried into the perforator to perform the step of perforating so as to form liquid medicine through-holes.
